# EUROPEAN PATENT SPECIFICATION

(11) **EP 2 201 124 B1**
(45) Date of publication and mention of the grant of the patent: **13.02.2013**
(21) Application number: 08806359.9
(22) Date of filing: 22.09.2008
(51) Int. Cl.: C12P 7/64, C12P 5/00

(54) **Method for producing biodiesel**
Verfahren zur Herstellung von Biodiesel
Procédé pour la production de biodiesel

(30) Priority: 21.09.2007 GB 0718490
(43) Date of publication of application: 30.06.2010
(73) Proprietor: Statoil ASA, 4035 Stavanger (NO)
(72) Inventor: KOTLAR, Hans, Kristian, N-4035 Stavanger (NO); SKARSTAD, Anita, N-4035 Stavanger (NO); MELLEMSAETHER, Evy, N-4035 Stavanger (NO); FREDRIKSEN, Geir, Remo, N-4035 Stavanger (NO)
(74) Representative: Icely, Dominic Michael
(86) International application number: PCT/GB2008/003204
(87) International publication number: WO 2009/037488

(56) References cited:
- DD-A- 249 712
- US-B1- 6 306 813
- SOLIMAN ET AL: "Molecular cloning and characterization of thermostable esterase and lipase from Geobacillus thermoleovorans YN isolated from desert soil in Egypt" PROCESS BIOCHEMISTRY, vol. 42, May 2007 (2007-05), pages 1090-1100, XP022138699
- THRONE-HOLST ET AL: "Identification of novel genes involved in long-chain n-alkane degradation by Acinetobacter sp. strain DSM 17874" APPLIED AND ENVIRONMENTAL MICROBIOLOGY, vol. 73, May 2007 (2007-05), pages 3327-3332, XP002450667
- SOOD ET AL: "Isolation and characterization of a potential paraffin-wax degrading thermophilic bacterial strain Geobacillus kaustophilus TERI NSM for application in oil wells with paraffin deposition problems" CHEMOSPHERE, vol. 70, October 2007 (2007-10), pages 1445-1451, XP022417982
- KASANA ET AL: "Isolation and identification of a psychrotrophic Acinetobacter sp. CR9 and characterization of its alkaline lipase" JOURNAL OF BASIC MICROBIOLOGY, vol. 48, June 2008 (2008-06), pages 207-212, XP002532097
- DU ET AL: "Perspectives for biotechnological production of biodiesel and impacts" APPLIED MICROBIOLOGY AND BIOTECHNOLOGY, vol. 79, April 2008 (2008-04), pages 331-337, XP019623601
- YAGIZ ET AL: "Biodiesel production from waste oils by using lipase immobilized on hydrocalcite and zeolites", CHEMICAL ENGINEERING JOURNAL, vol. 134, 2007, pages 262-267, XP022223954,

## Description

This invention relates to process for the production of biodiesel, in particular to a process for reducing hydrogen consumption during biodiesel formation using microorganisms from the species Geobacillus or Acinetobacter to hydrolyse the glycerides present in the biocomponent.

There is increasing World concern about climate change and hence carbon dioxide emissions. In Europe at least, measures are now being taken at the highest level to try to reduce carbon dioxide emissions in all walks of life. This means a heavier reliance on renewable energy sources such as solar and wind power, increasing taxation on energy inefficient products and more investment in harnessing the power of the sea. A further quickly developing field is biofuels, especially for vehicles.

The biofuels directive of 2003 (Directive 2003/30) established an indicative target value of 5.75 % market share for bio-fuels at the end of 2010. The reference value is based on the energy content of the fuel, and each member state is to set national targets. In the Energy and Climate Package and the related Renewable Energy Roadmap published on 10 January 2007, the European Commission proposes a minimum binding 10% target of biofuels for vehicle use to be reached in the EU by 2020.

The use of flexifuel vehicles, which run on alcohol, in particular ethanol (and also methanol), are already well known. Ethanol can be produced from sugar cane and is frequently used in blends with gasoline to form a biofuel (E5, E85). Methanol is however toxic and is not an ideal material for the mass market, its use currently being confined therefore to racing vehicles.

The industry has therefore being considering ways in which other biomasses can be incorporated into both gasoline and diesel.

Components for diesel fuel can be prepared in various ways but the major diesel components are manufactured using mild hydrocracking processes where a mineral oil feed is treated with a Ni/Mo or Co/Mo type catalyst (a hydrotreating step) before being subjected to isomerisation in the presence of a zeolite type catalyst. Such a process is well known and carried out industrially around the globe. The hydrotreating step involves is designed to remove contaminants such as sulphur and nitrogen from the oil and also serves to hydrogenate certain compounds in the mineral oil, such as condensed ring aromatics, which are unacceptable in the final fuel. The isomerisation step causes the hydrocarbons to rearrange or crack so that as many hydrocarbon components as possible have a boiling point within the diesel fuel range (typically 221 to 360°C). In some situations, it may only be the hydrotreating step which is carried out.

To introduce a biodiesel element into a diesel fuel, it is known to add a conventional mineral oil to a biomass component. Vegetable oils such as rape seed oils are currently the most common biomass added to biofuels. In US 2006/0186020 the hydroconversion of a mixture of vegetable oil and mineral oil is described to form a biodiesel material. A similar disclosure is made in WO2004/022674. In must be remembered however that hydrotreating and isomerisation processes described in these and other state of the art documents were originally optimised to deal with mineral oil feeds alone. The catalysts used were not intended to be used in the presence of biomass and the conditions of mild hydrocracking have been optimised for mineral oil treatment.

Moreover, unlike mineral oil feedstocks, biomass feeds contain high levels of fatty acid esters, especially glycerides. In order to convert a glyceride into a form suitable for use in a diesel engine, as a first step it is necessary to hydrolyse the ester bond and remove the glycerol left behind. This is currently achieved in a conventional hydrotreating step where, as noted above, the feedstock is hydrogenated. During hydrogenation ester bonds can be cleaved and the resulting materials (glycerol and fatty acids) reduced to their corresponding hydrocarbons. Hydrogenation is, however, an expensive and energy intensive process. It requires a complex and expensive reactor and uses an expensive and dangerous gas. Hydrogenation catalysts are also expensive and require regular regeneration. Anything that can be done to reduce the hydrogenation requirement therefore has important economic and safety ramifications in the industry.

It must also be reiterated that hydrotreating reactors are designed to hydrotreat a mineral oil feed which does not contain esters. The hydrogen requirements to hydrolyse and then hydrotreat are very different from conventional hydrotreating.

Yagiz et al., Chemical Engineering Journal 134: 262-267 (2007) disclose biodiesel production from waste cooking oils by using lipases.

The present inventors have now realised that for a biological source of fuel such as an animal fat, fish oil or vegetable oil, the necessary manipulation of the feedstock into a form suitable for use in diesel can be started using certain microorganisms. The primary constituent of biomass of interest to the biodiesel formulator are the fatty acid esters compounds. The inventors have realised that microorganisms that can target these compounds and hydrolyse the esters thereof can convert a low cetane rated biomass into a higher cetane rated fuel component with potential for mixing with a mineral oil component directly or, more commonly, with potential for entering a conventional hydrotreating or isomerisation process.

Hydrotreatment of an enzymatically pretreated biofeedstock will thus resemble a conventional refinery hydrotreating unit operation using a commercially available hydrotreating catalyst as is known in the art. However, it is believed that hydrogenation of enzyme pretreated feedstock will produce slightly different product composition depending on the action of the particular enzymatic pretreatment.

Unlike the hydrogenation procedures used for biodiesel today, in some cases no CO, CO₂ or water will be produced as hydrogen will be acting purely to hydrogenate double bonds and not to hydrolyse esters. This will have favourable effects on catalyst activity and stability as well as refinery hydrogen economy and the make up hydrogen compressor size. Furthermore, it will be easy to integrate a pre-treatment process into a existing hydrotreatment unit because no change to the existing hydrotreatment reactor is required. A separate biooil hydrotreating process unit can thus be avoided.

Moreover, by hydrolysing using microorganisms, the process of the invention does not require high temperatures or expensive catalysts to effect hydrolysis, there is no requirement for high pressure and the use of expensive and dangerous hydrogen is avoided. This has major economic impact for the petrochemical industry.

In addition, as the microorganisms are not sensitive to the nature of the feedstock, this method allows the biodiesel manufacturer to prepare a biodiesel component from a mixture of different bio sources. It is quite possible therefore for the microorganisms to work on a substrate formed from a mixture of vegetable, animal and/or fish sources simultaneously. Given that to achieve the EU's desired biocontent in diesel fuel across Europe would require enormous amounts of farm land to be devoted to preparing biofuel, any way of maximising biocontent has huge benefit in terns of minimising environmental impact. The process of the invention will preferably be running under thermophilic conditions, e.g. around 60°C. This high temperature is important for liquefying animal fat and at this temperature enzymatic reactions can be run in such a way that undesired contamination and side reactions are avoided.

Thus, viewed from one aspect the invention provides a process for the formation of biodiesel comprising:
(I) obtaining a biooil containing at least one fatty acid glyceride;
(II) contacting the biooil with at least one microorganism from the species Geobacillus or Acinetobacter capable of hydrolysing at least one ester bond of said at least one glyceride to form a hydrolysed biooil;
(III) directly or indirectly using said hydrolysed biooil in biodiesel.

Viewed from another aspect, the invention provides a process for the formation of biodiesel comprising:
(I) obtaining a bio oil containing at least one fatty acid glyceride;
(II) contacting the biooil with at least one microorganism from the species Geobacillus or Acinetobacter capable of hydrolysing at least one ester bond of said at least one glyceride to form a hydrolysed biooil;
(III) hydrotreating said hydrolysed biooil; optionally
(IV) isomerising the hydrotreated biooil; and
(V) using the product of step (III) or, if carried out, step (IV) in biodiesel.

### Definitions

By biodiesel is meant that the diesel contains a component which is derived from a renewable biological source, e.g. a plant oil, marine oil or animal oil. Preferably the biodiesel will also contain a mineral oil component.

By biooil is meant an oil derived from a renewable biological source such as a plant, animal or fish. The term biooil covers animal fats which might be solid at room temperature but which can be melted upon gentle heating. Preferably, the biooil will not have been refined.

By hydrolysing at least one ester bond of said at least one glyceride to form a hydrolysed biooil is meant that at least one ester linkage of the glyceride (preferably all ester linkages) are hydrolysed to give a fatty acid and glycerol.

By hydrolysed biooil is meant one in which the at least one ester bond of the at least one glyceride has been hydrolysed to its constituent fatty acid and glycerol residue.

### Biooil

The biooil of use in the invention can be obtained from any convenient source but it one which contains at least one glyceride. The biooil may be derived from a plant, animal of fish.

Useful plant oils include soya oil, rapeseed oil, olive oil, sunflower oil, colza oil, canola oil, hempseed oil, linseed oil, mustard oil, palm oil, peanut oil, castor oil and coconut oil.

Useful animal oils (which term includes animal fats) include tallow oil and lard.

Fish oils are those oils which are derived from marine sources such as fish, micromarine organisms (krill and the like) or marine mammals such as seals. Whilst it is preferred if the fish oil derives from a sea based organism, in this invention, the term fish oil is intended to cover freshwater sources of fish oils.

Also within the term biooil are recycled oils e.g. those used in the food industry and the like.

In a highly preferred embodiment the biooil comprises fish oil.

It is also within the scope of the invention for a mixture of biooils to be employed, e.g. a mixture of plant oil and marine oil.

Biooils are often characterised by their free fatty acid content (FFA). Oils with a FFA of less than 1.5 % are regarded as refined and those with an FFA of greater than 20% are regarded as high FFA materials, typically animal fats. The present invention is of particular utility with oils having an FFA of at least 5%, preferably at least 10%.

The biooil is preferably not one which has undergone refinement. Many vegetable and fish oils are refined to provide a source for allegedly health improving fatty acids such as omega-3. Such refined fish oils are of little interest in this invention as the cost of the refined oil is too high for use in the great bulk required to manufacture a useful amount of biodiesel. Thus, the oil used should preferably be unrefined and used essentially in the form it is isolated in from the natural source. Marine oil, especially fish oil, is a known waste product from the fish industry and this waste product, in unrefined state provides the ideal starting material for the process of the invention.

However, some biooils might contain compounds which could be considered poisons to the catalysts involved in the hydrotreating and isomerisation processes. It may therefore be necessary to remove such compounds. Vegetable oils, for example, might need degummed.

### Glyceride

The biooil will contain at least one fatty acid ester in the form of a glyceride. The glyceride will typically be a triglyceride although could also be a mono or diglyceride. The fatty acid portion of the glyceride may be, independently of formula (I):

CH₃(CH₂)ₙ-(CH=CH-CH₂)ₘ-(CH₂)₅-COO (I)

wherein n, and s are integers, e.g. of 1 to 10 and m is 0 to 10. Preferably, the number of carbon atoms in the fatty acid component will be at least 10, e.g. 12 to 40.

It will be appreciated that most natural biooil sources will contain a variety of different glycerides with different fatty acid portions. Preferably therefore the biooil contains a plurality of fatty acid glycerides which can hydrolysed by the microorganisms.

### Microorganisms

The microorganism of use in the invention is one from the species Geobacillus or Acinetobacter, which is capable of hydrolysing at least one ester bond in a glyceride compound so as to yield glycerol (or a reduced glyceride) and fatty acid. Preferably, the microorganism will hydrolyse all ester bonds present to reduce the glyceride to fatty acids and glycerol.

Said microorganism may be capable of hydrogenating double bonds in the fatty acid chains of the glyceride. Many naturally occurring glycerides contain double bonds along the length of the carbon backbone and it is preferred if these bonds can be saturated using the microorganism. This further reduces the hydrogen requirement of the overall biodiesel formation process.

Said microorganism may be capable of cracking the hydrocarbon chains of the fatty acids to make smaller chains which will improve the cetane rating of the biodiesel.

Even more preferably, said microorganism may be capable of reducing the formed fatty acid compounds into aldehyes, alcohols or to a hydrocarbon. It will be appreciated however that some reduction of acid groups might also occur during the hydrogenation.

In order to ensure that the necessary hydrolysis of ester linkages in the glyceride (and optional reduction of resulting acids) takes place it is preferred, however, to use a mixture of different microorganisms. Preferably, the microorganism composition will contain at least 2 and preferably at least 3 different microorganisms. In some embodiments there may be at least 5, e.g. at least 10, preferably at least 20, especially at least 100 microorganisms added to the biooil.

By different microorganisms is meant that there are at least two distinguishable microorganisms present. There may be microorganisms from a different genus, different species, different strains or even different mutant strains of microorganism. Differences in microorganisms can be detected using genetic techniques such as ribosomal RNA analysis.

Preferably all microorganisms are selected from the group consisting of Bacillus sp., Thermus sp., Pseudomonas sp., Geobacillus sp., Arthrobacter sp., Sphingomonas sp., Mycobacterium sp., Burkholderia sp., Acinetobacter sp., Thermovirga sp., Archaeoglobus sp., Thermosipho sp., Symbiobacterium sp., Methanosaeta sp., Epsilon proteobacterium sp., Syntrophus sp., Nocardioides sp., Deferribacter sp. and Chloraflexi sp., whereby at least one is selected from Geobacillus sp. or Acinetobacter sp.

In a further preferred embodiment, the microorganism may also be capable of or is mixed with a further microorganism capable of chain-shortening alkanes.

Examples of microorganisms capable of chain-shortening alkanes include *Bacillus sp., Geobacillus sp., Acinetobacter sp., Methanosaeta* sp. and in particular *Acinetobacter venetianus, Bacillus thermoleovorans, Bacillus aeolis* and *Geobacillus thermodenitrificans. Use of Pseudomonas sp*. will result in n-alkane degradation and reduced viscosity, e.g. *Pseudomonas aeruginosa*. Moreover, *Thermus brockii* is capable of degrading hexadecane (see Geitkenhauer et al., Water Sci Technol 47: 123-130(2003)).

Highly preferred microorganisms for use in the invention include Acinetobacter. *venetianus* and in particular strain 6A2 (DSM17874) and mutants thereof (TMT1, TMT2, TMT3, WT and MAV1), *Geobacillus pallidus, Geobacillus kaustrophilus, Geobacillus toebli and Geobacillus thermoglucosidasius.* Acinetobacter, *venetianus has been* found not only to hydrolyse the ester linkages in a glyceride molecule but also to reduce alkane chain length making it an ideal microorganism for use in this invention. Strain DSM17874 has been deposited at the DSMZ both in the open collection (deposit number DSM17874 by Mimmi Throne-Holst) and under the conditions of the Budapest Treaty on 20 June 2007, by Statoil ASA, under accession number DSM19446.

Viewed from another aspect therefore the invention provides a process for the production of biodiesel comprising:
(I) obtaining a biooil containing at least one fatty acid glyceride;
(II) contacting the biooil with Acinetobacter. venetianus to form a hydrolysed biooil;
(III) directly or indirectly using said hydrolysed biooil in biodiesel.

It is especially preferred that the biooil is contacted with microorganisms selected from the species *Bacillus thermoleovorans, Thermus brockii, Acinetobacter venetianus, Deferribacter desulfuricans, Tlzermosipho geolei, Thermosipho africanus, Symbiobacterium thermophilium, Thermovirga lienii, Sphingomonas stygia, Sphingomonas aromaticivorans, Sphingomonas subterranean, Sphingomonas yanoikuyae, Pseudomonas putida, Burholderia sp.* and *Archaeoglobus fulgidus* whereby at least one is selected from the species Geobacillus or Acinetobacter. Particular deposited strains that can be used include *Bacillus thermoleovorans* AB034902 (Genbank), *Bacillus aeolis* AY603079 (Genbank), *Pseudomonas aeruginosa* AM087130(Genbank), *Geobacillus thermodenitrificans* DQ243788(Genbank), *Geobacillus subterraneous* DQ355385(Genbank), *Sphingomonas stygia* DSMZ12445, *Sphingomonas sp* DSMZ 7526, *Sphingomonas sp* DSMZ 11094, *Sphingomonas aromaticivorans* DSMZ 12444, *Sphingomonas subterranean* DSMZ 12447, *Sphingomonas yanoikuyae* DSMZ 6900, *Pseudomonas putida* NCIMB 9815, *Pseudomonas putida* NCIMB 9816, *Pseudomonas putida* NCIMB 10015, *Methanosaeta sp.* AJ 133791, *Epsilonproteobacteria* AY 570641, *Syntrophus aciditrophicus* CP 000252, *Nocardioides sp.* D 87974, *Deferribacter desulfuricans* AB 086060, *Chlorflexi sp.* AB 074961, *Thermovirga lienii* DQ 071273, *Archaeoglobus fulgidus* DQ 131905, *Thermosipho geolei* AJ 272022, *Acinetobacter venetianus* ATCC 31012 and *Symbiobacterium sp.* AB 052392.

Rather than producing a microorganism inoculation composition by mixing individual microorganisms on site, it is possible and indeed preferable to use microorganism cocktails from or developed from naturally occurring microorganism communities, e.g. microorganism communities from subterranean hydrocarbon reservoirs, from oil shales, bitumen and asphalt sources, waste water treatment, earth sediments or, especially oil contaminated sand. Naturally occurring microorganism cocktails comprising at least one of *Geobacillus pallidus, Geobacillus kaustrophilus, Geobacillus toebli and Geobacillus thermoglucosidasius* are especially preferred.

Likewise appropriate microorganisms may of course be produced by mutagenesis or by genetic engineering.

The temperature at which the microorganisms and substrate should be inoculated can vary over a wide limit e.g. 10 to 100°C. Preferably the process should take place between 20 and 80°C. It some embodiments, however, microorganisms may be required to work at temperatures greater than ambient, e.g. at temperatures of at least 50°C to ensure that the biooil substrate is in liquid form. Some animal fats are solids until around this temperature and it may be necessary therefore to operate at slightly elevated temperatures to ensure that the biooil is a liquid. In a further preferred embodiment therefore the hydrolysis reaction (step (II)) takes place at a temperature of at least 30°C, preferably at least 40°C, especially at least 50°C, most especially at least 55°C.

Ideally the microorganisms will operate in an aerobic environment but it is of course possible to operate anaerobically if this is necessary to maximise microorganism function.

It is preferred if the mixture of substrate and microorganisms is agitated (e.g. shaken, stirred or sonicated) to maximise hydrolysis potential.

The biooil can be exposed to the microorganisms over a broad timer period typically of the order of 1 day to two weeks, e.g. 7 days. An industrial process, may however be run continuously and adjusted to have an appropriate residence time.

It may also be necessary to add to the biooil substrate some form of mineral medium which provides the microorganisms with nutrients essential for their growth. The addition of such a medium is well known in the art.

Particular microorganism combinations can be devised to treat a biooil substrate of interest. The nature of the compounds in each substrate is slightly different and some microorganism may perform better on animal fat substrates than plant substrates and so on. Identifying a particular combination of microorganisms can be readily achieved by a simple screening process.

*A. venetianus* 6A2 and mutants thereof (TMT1, TMT2, TMT3, WT and MAV1) have been found to be particularly useful in hydrolysing glycerides linkages in animal and fish oils.

Once the microorganisms have hydrolysed the glycerides in the biooil the glycerol can be removed in an aqueous wash and the resulting oil phase can be used directly in biodiesel. Preferably however, it would be normal for the oil phase mixture to be transferred to conventional hydrotreating equipment for further manipulation into a diesel fuel.

The hydrotreating process cannot function if the oil is added to the hydrotreator unit in solid form. It is required therefore that if the hydrolysed material is solid, that the oil be melted before being added to the cracker. Most biooils will, of course, be in liquid form in their natural state but some, in particular animal oils may present as solids and need to be melted. Melting can be achieved simply by heating the oil to a temperature greater than its melting point, typically 60°C.

The hydrolysed biooil component will typically be added to the hydrotreatment apparatus along with a mineral oil component, i.e. the hydrotreater will not simply operate on bio sources it will also hydrotreat mineral oils as is well known in the art. The biooil can thus be mixed with a mineral oil and added together with the mineral oil to the hydrotreator or mixing could occur in the reactor itself and hence the mineral oil and biooils could be added via separate addition points. It will also be appreciated that the process of the invention is likely to be carried out continuously in that new feed will be added to a reactor in which there is already a hydrotreating reaction occurring. This also falls within the scope of the invention.

The relative amounts of biooil added relative to the mineral oil feed can vary over a wide range as long as the resulting biodiesel product can function successful in the combustion engine. Preferably however, the amounts range from 0.5 to 50 wt% bio oil, preferably 1 to 20 wt% biooil, especially 3 to 15 wt% biooil, e.g. 5 to 10 wt% biooil.

By mineral oil component (also termed the hydrocarbon feedstock herein) is meant a component which is derived from crude oil. The mineral oil/hydrocarbon feedstock on which the process above operates can be any suitable feed, e.g. any distillate oil. Preferably however, the feed comprises light and/or heavy gas oils, (especially straight run light or heavy gas oils of crude oil), vacuum distillates, vacuum gas oil, coker gas oil, light cycle oil and materials which are produced during coking, e.g. delayed coking or fluid catalytic cracking. The use of light gas oil or heavy gas oil, especially straight run light gas oil or straight run heavy gas oil is especially preferred.

The boiling point of the hydrocarbon feedstock may be in the range from 150 to 550°C, in particular 250 to 450°C, preferably 280 to 410°C. The density of the hydrocarbon feedstock may be greater than 845 kg/m³, e.g. greater than 860 kg/m³.

The sulphur content of the hydrocarbon feedstock may be at least 500 ppm, preferably at least 750 ppm, especially at least 1000 ppm (by weight).

The nitrogen content of the hydrocarbon feedstock may be at least 150 ppm, preferably at least 200 ppm (by weight).

The initial cetane index (D4737/90) for the hydrocarbon feedstock may be 45 to 51.

The hydrocarbon feedstock may comprise at least 20% aromatics, e.g. at least 25 % aromatics, such as 25 to 70 wt% aromatics, e.g. at least 28 wt% aromatics, such as at least 35 % aromatics. The hydrocarbon feedstock may comprise up to 20 wt% monoaromatics, up to 10 wt % diaromatics and up to 5 wt % triaromatics.

Once mixed with the bio oil, the mixed feed may have a density of at least 0.860 kg/l, preferably at least 0.865 kg/L. The cetane number may be in the range 45 to 50. Thus, the mixture of marine oil and mineral oil has a lower cetane number but higher density than the mineral oil alone.

The process of the invention can be carried out in a conventional hydrotreating process layout. The process can occur in a single step, i.e. hydrogenation, desulphurisation and isomerisation of the feedstock can all occur in the same reaction step or it could be carried out in more than one step.

The hydrotreating catalyst system can be present in a single bed or multiple beds. In a further embodiment, the zeolite catalyst system can be present in one bed with a hydrotreating catalyst present in a separate, preferably earlier bed. The person skilled in the art is able to manipulate the reactor set up to suit his needs.

Hydrogen is added to the hydrotreating step to effect hydrogenation and desulphurisation of the hydrocarbon feedstock.

An ideal reactor set up may involve addition of the marine oil and hydrocarbon feedstock with hydrogen rich treat gas to the reactor, i.e. it is preferred if addition of the hydrogen and feedstock occur through the same reactor inlet. Whilst it would be possible to feed these separately, mixing them is preferred. In a further preferred embodiment, the feed or feeds to the reactor are preheated, preferably to a temperature similar to that of the reactor at the inlet point. Thus, if the reactor temperature is 350°C at the inlet point, then the feed should be heated to approximately this temperature prior to its addition to the reactor.

Preheating of the feed can be achieved using an external heat source but ideally it is effected by heat exchange with the reactor effluent stream. Should heat exchange not heat the feed sufficiently, external heating means can be used to supplement the preheating process.

As the reactor feed passes through the reactor and hence over the catalyst in the reactor, it is preferred if the temperature increases through the reactor, i.e. from inlet to outlet. The temperature increase through the reactor may be at least 20°C, e.g. at least 30°C.

Where the reactor contains a plurality of catalyst beds, i.e. the feed passes over more than one catalyst bed between the inlet and reactor outlet, it is possible to cool the reactor between beds by the introduction of a quench gas, typically hydrogen. This not only cools the reactor but provides further hydrogen for hydrogenation.

The hydrotreating catalyst used can be one which is conventional in the art, e.g. one based on metals from groups VIB and VIII. Preferred combinations are based on Ni or Co with Mo or W. NiMo or CoMo are especially preferred.

Suitable zeolite catalysts for use in the isomerisation state are also well known in the art with beta zeolites being commonly used.

Once the desulphurisation, hydrogenation and isomerisation have occurred the reactor effluent may be cooled and mixed with wash water before further cooling, e.g. by air cooler or other heat exchange, to the required separator temperature. In the separator sour water, reacted feedstock and gas may be separated. Sour water may routed back to the sour water system, the gas (hydrogen) may be recycled to the reactor and the reacted feedstock is sent to a product stripper where light products, such as hydrocarbon gases and naphtha, are sent overhead and the gasoil product is taken out as the bottom product.

The gas is typically sent to H₂S recovery, the naphtha to further processing or to product tankage, and the gasoil product is sent to product tankage for subsequent use in diesel fuel.

The process of the invention is carried out under particularly mild conditions and this is a further aspect of the invention. In particular low pressures can be employed. Low pressures mean a more economic process and are highly desirable. The process of the invention preferably occurs at a temperature of from 250 to 500°C, preferably 300 to 450°C, especially 300 to 400°C. The pressure is less than 100 barg but preferably at least 10 barg, e.g. 40 to 100 barg, such as 45 to 60 barg. Barg is gauge pressure, i.e. the pressure measured in bars on a pressure gauge (thus relative to the ambient pressure).

Suitable hydrogen to feedstock ratios may be at least 75 Nl/l, e.g. 100 to 1500 Nl/l, preferably 150 to 500 Nl/l. (The unit Nl/l represents normal litre hydrogen at 0°C and 1 atm pressure per litre feedstock). The liquid hourly space velocity (LHSV) may be between 0.3 to 5/h, e.g. 0.5 to 2/h.

The catalyst can be regenerated by conventional techniques, e.g. by burning off any coke which forms on the catalyst composition.

The product of the process as hereinbefore defined has a much lowered sulphur content relative to the feedstock. Sulphur contents in the hydrocarbon product which exits the ring opening reactor can be less than 50 ppm, e.g. less than 20 ppm, especially less than 10 ppm. The amount of sulphur present in the hydrocarbon product can be reduced further by increasing the operating temperature.

The invention also effects denitrogenation of the feedstock. Levels of less than 10 ppm in the product can be achieved, e.g. less than 2 ppm. For straight-run HGO as an example, the nitrogen levels in the feedstock may be of the order of 250 ppm which reduces to less than 2 ppm after hydrotreating.

After the process of the invention, the boiling point of the majority (i.e. at least 50 wt%) of the hydrocarbon product, i.e. the ring opened feedstock, should be in the range from 150 to 360°C, preferably at least 60 wt%.. Preferably, at least 90% of the product, especially 95% of the product is formed from hydrocarbons having a boiling point below 395°C, preferably below 380°C, especially below 360°C.

The amount of naphtha component (i.e. liquid components boiling below 150°C) produced during the process should be less than 40% wt, preferably less than 30 % wt, especially less than 15% wt, most especially less than 10 wt% of the product. Such naphtha can of course be isolated and used as is known in the art.

The amount of hydrocarbon gas produced (i.e. C1-C4 fraction) is also minimised, e.g. to less than 5 wt%. Again, these gaseous products can be isolated and used as is known in the art. The inventors have surprisingly found that the addition of marine oil to a hydrotreating process yields much higher levels of propane than are reported with mineral oil feeds alone. Propane is not a constituent of diesel so this needs to be isolated (by well known conventional separation techniques) from the diesel fraction but can be used in many industrial processes either as a fuel, diluent or to make propene.

The density reduction achieved using the process of the invention from feedstock to ring opened product is preferably at 0.10 kg/L. This reduction is preferably achieved relative to the formed product even after the naphtha and gas fractions are removed..

Surprisingly, despite the combination of bio oil and mineral oil having a higher density than the mineral oil alone, the resulting treated feedstock can have a lower density than that of the similarly treated mineral oil. The overall density reduction is thus much more marked for the mixed feed of the invention.

The density of the hydrocarbon product is preferably less than 855 kg/m³ especially less than 852 kg/m³. Whilst the density can be reduced further by increasing the temperature of the process this also results in increased naphtha production.

The amount of monoaromatics in the product stream can be reduced to less than 15 wt%, the amount of diaromatics to less than 2 wt% and the amount of triaromatics to less than 0.5 wt% using the process of the invention, especially for a heavy gas oil feedstock. The total aromatic content may therefore reduced to less than 17.5 wt%.

In addition, the naphthenes content of the product (i.e. cyclic aliphatic hydrocarbon content) may be greater than 45 wt%.

The cetane number of the cracked product is preferably greater than 51, especially greater than 53. Surprisingly, the inventors have found that the addition of the marine oil to the mineral oil gives rise to a diesel component with a higher cetane rating than that produced using the mineral oil alone. This is despite the mineral oil feed having a higher rating than the mixed feed. As higher cetane rating means a cleaner fuel and hence lower emissions, the invention has the double benefit of using renewable biomass as a fuel source and forming a cleaner burning fuel.

The product can be fractionated or passed to further reactors for further treatment as is desired. It is also possible to recycle heavy fractions back into the hydrocracker. Preferably however, the hydrocarbon product stream, after naphtha and gas removal, is suitable for direct use in automotive diesel.

It is also envisaged that the ester hydrolysis reaction described herein could be used to allow inclusion of a biooil component in gasoline or other biofuel.

The invention will now be described with reference to the following non limiting examples and figures.
Figure 1a shows the LC-MS for a control fish oil not subjected to microorganism treatment.
Figure 1b shows the LC-MS for the same fish oil after treatment with A. *venetianus* 6A2 (TMT3) for 7 days at 30°C.
Figure 2 shows a 3-D score plot of triglyceride levels in unrefined fish oil treated with inoculum from masterplate mix L002 (red), L004 (green), L018 (blue) and L019 (yellow) for 7 days at 60°C. For each of the tests a negative control with no inoculum is run simultaneously. This is represented by the black spots..
Figure 3 shows a 3-D LC-MS of fatty acid levels in unrefined fish oil treated with inoculum als described for figure 2.
Figure 4 shows a 3-D score plot of triglyceride levels in refined fish oil treated with inoculum as described for figure 2.
Figure 5 shows a 3-D LC-MS of fatty acid levels in refined fish oil treated with inoculum as described for figure 2.
Figure 6 shows a 3-D score plot of triglyceride levels in animal (tall) oil treated with inoculum as described for figure 2.
Figure 7 shows a 3-D LC-MS of fatty acid levels in animal treated with inoculum as described for figure 2.
Figure 8 shows a 3-D score plot of triglyceride levels in soya oil treated with inoculum as described for figure 2.
Figure 9 shows a 3-D LC-MS of fatty acid levels in soya oil treated with inoculum as described for figure 2.

### Examples

Cultures of masterplates and *Acinetobacter venetianus* 6A2 (including mutants) were prepared according to the following protocols:

### Example 1:

### Protocol

*A. venetianus* 6A2 (DSM19446) and mutants thereof (TMT1, TMT2, TMT3 and MAVI) were grown aerobically at 30°C in Luria Broth (LB) with antibiotics as appropriate (kanamycin 25 mg/L, apramycin 50 mg/L, chloramphenicol 30 mg/L). Eventually, the bacteria were harvested by centrifugation, washed with mineral medium (MM) and resuspended in MM at a final concentration of 1 O.D. The latter named inoculum.

### Example 2

### Masterplate Protocols

Four different masterplates were cultivated aerobically in a mixture of mineral medium (MM), yeast extract (YE) and acetate (Ac) for 48 hours at 60°C. Eventually, the bacteria were harvested by centrifugation, washed with MM and resuspended in MM at a final concentration of 1 O.D., the latter named inoculum.

The masterplates are described in table 1

**Table 1**

| **96-well plate** | **Source** | **Sample type** | |
|---|---|---|---|
| MM Ac YE-Masterplate L002 | 1. Samples from Crete | 1. Sand /sediments polluted with waste oil / fuel | No facultative anaerobic organisms found among the isolates from the plate |
| | 2. Samples from Trondheim compost | 2. Common garden compost, Trondheim | |
| MM Ac YE Masterplate L004 | 1. Water treatment plant NL | 1. Water samples | A total of 11,5 % of the organisms are facultative anaerobic organisms, these are isolates from water samples-NL (constitute 64 % of the facultative anaerobic organisms in L004) and the Mexico Gulf (constitute 36 % of the facultative anaerobic organisms in L004) |
| | 2. Tar sand, Canada | 2. Oil sand | |
| | 3. Road samples, N.Trl. | 3. Samples from old asphalt roads | |
| | 4. Gulf of Mexico | 4. Various samples of sediments | |
| | 5. Tepee Butte, USA | 5. Samples of sediments, clay and water | |
| MM Ac YE Masterplate L018 | Water treatment plant NL | Water samples | 47 % of the isolates are facultative anaerobic organisms |
| MM Ac YE Masterplate L019 | | 1. Oil sand | 6 % of the isolates are facultative anaerobic organisms |
| | 1. Tar sand; Canada | 2. Bio mud | 100 % of the isolates are sporogenous (isolatedafter enrichment culture-boiling) |
| | 2. Water treatment plant, Mongstad | 3. Sand, sediment samples collected near by an oil refinery | |
| | 3. Australia | | 50 % of the isolates are isolated from the enrichments with Zuata oil as the sole C-source |

| | | | |
|---|---|---|---|
| Masterplate L002 contains *Geobacillus pallidus,* Masterplate L004 contains *Geobacillus pallidus, Geobacillus kaustrophilus* and *Geobacillus toebli* Masterplate L018 contains *Geobacillus thermoglucosidasius.* | | | |

### Example 3

### Inoculation with Biooil

MM, vegetable or animal oil and inoculum were added to baffled 250 ml shaking flasks at 48 ml, 12 ml and 1 ml, respectively. MM contains a minimum of minerals, metals and vitamins essential for bacterial surveillance. The carbon source is supplied by vegetable or animal oil triglycerides. To get accustomed to animal and vegetable fat as a sole carbon source the bacteria were induced with 2 % oil. After 48 hours the oil content was raised to 20 %. As a reference, a negative control lacking inoculum was included within each experimental series.

All experiments were conducted in a shaking incubator at 150 rpm, 60 % humidity and appropriate temperature (30 or 60 °C) depending on the bacterial properties.

The water phase and the oil phase were harvested at the seventh day and prepared for LCMS and pour point analysis. The results are presented in the attached figures.

### Discussion

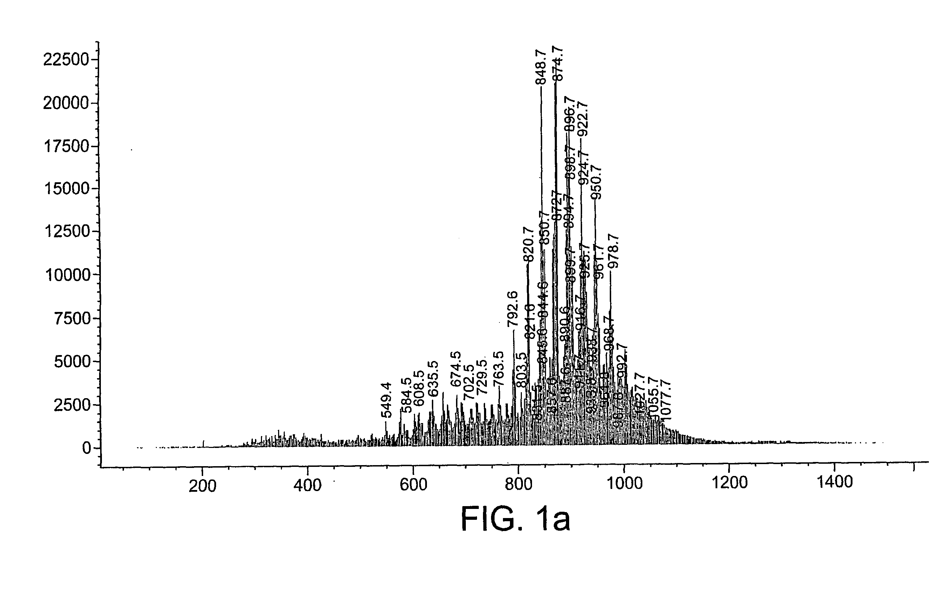

Figure 1a shows the LC-MS for a control fish oil not subjected to microorganism treatment.

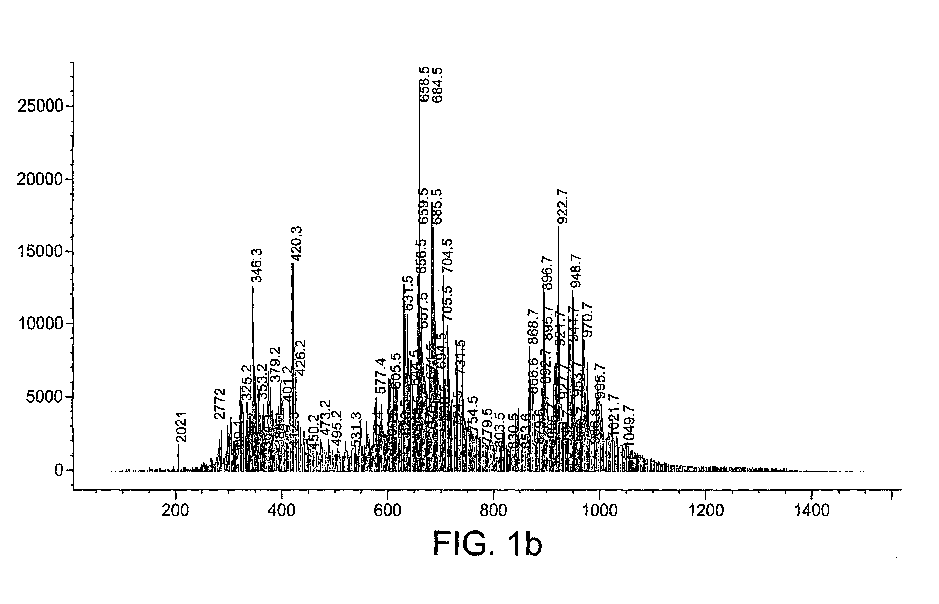

Figure 1b shows the LC-MS for the same fish oil after treatment with A. *venetianus* 6A2 (TMT3) for 7 days at 30°C. In figure 1b it can be seen that the triglyceride has been bioconverted into di-and monoglycerides. Fatty acids and/or fatty acid esters have therefore been generated.

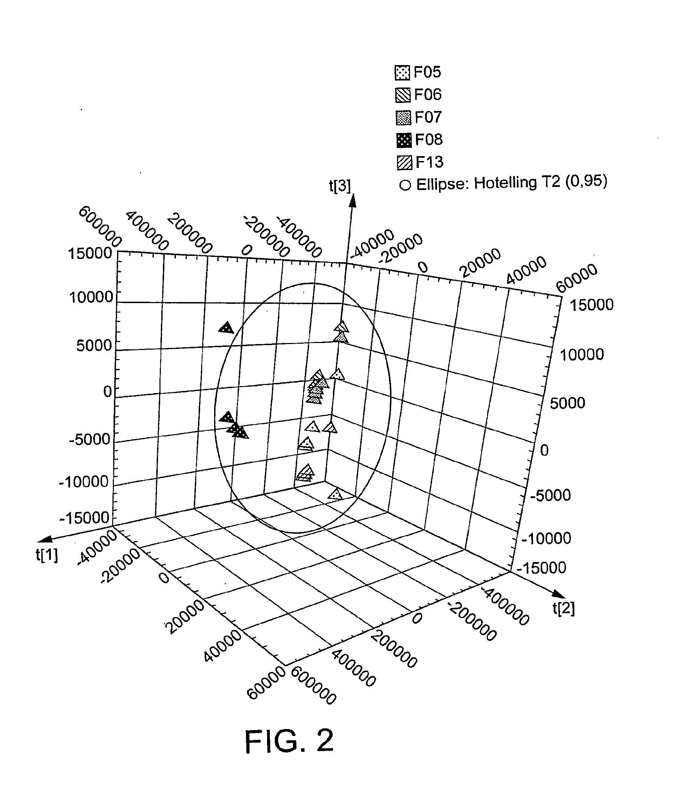

Figure 2 is a 3D score plot of the bioconversion of raw fish oil and shows the differences in triglycerides after treatment in comparison to a control. In the figures inoculi corresponding to L002 are red, L004 are green, L018 are blue and L019 are orange. Inoculums from masterplates L019 and L004 give the largest change in triglyceride levels compared to the control.

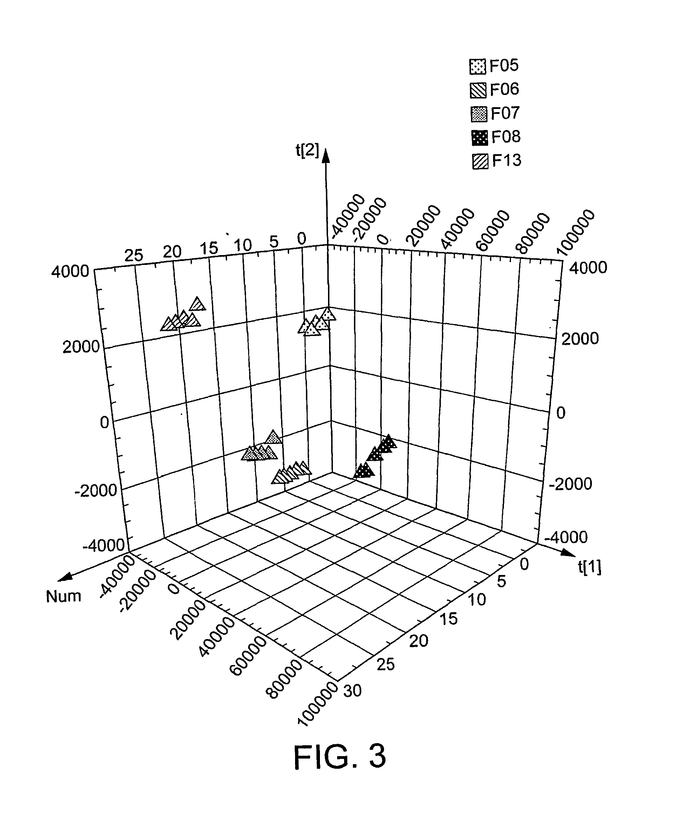

Figure 3 is an LC-MS spectrum at negative ionization showing changes in fatty acid composition. Inoculi corresponding to masterplates L019 and L004 prove to give the highest effects.

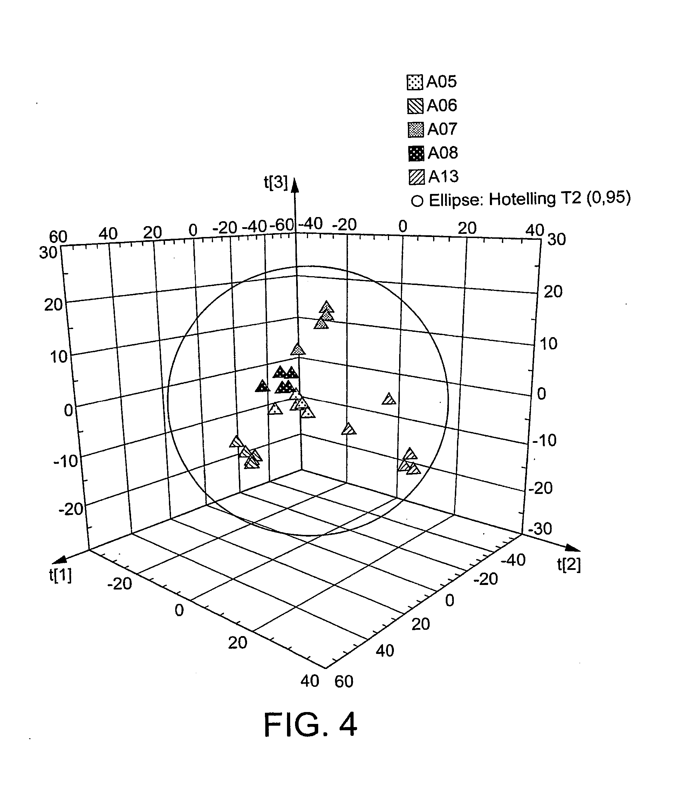

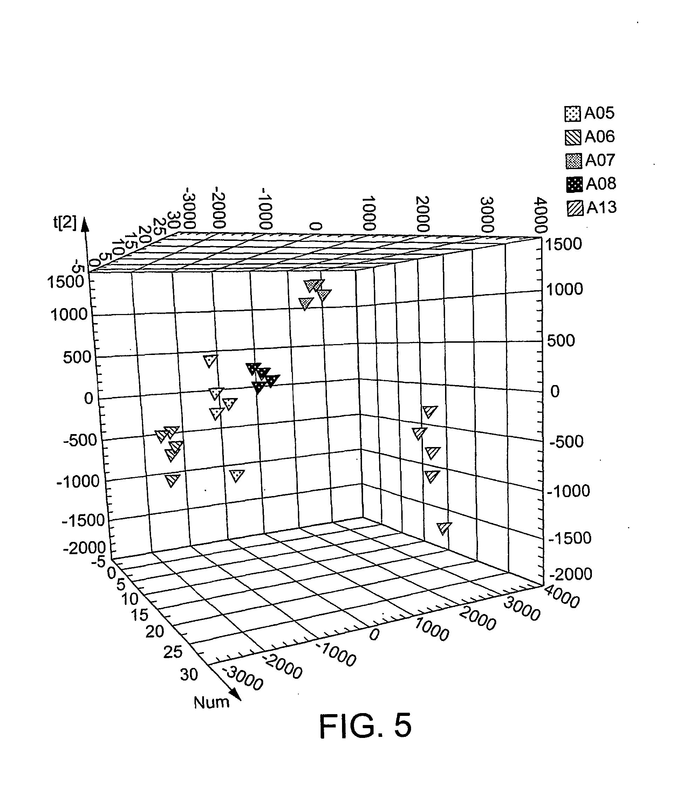

Figure 4 is a 3-D score plot of the bioconversion of refined fish oil and gives the difference in triglyceride composition of bioconverted refined fish oil compared to a control. The largest conversion are seen with inoculi corresponding to L004, L018 and L019. The LC-MS spectrum of fatty acids give the corresponding effect in figure 5.

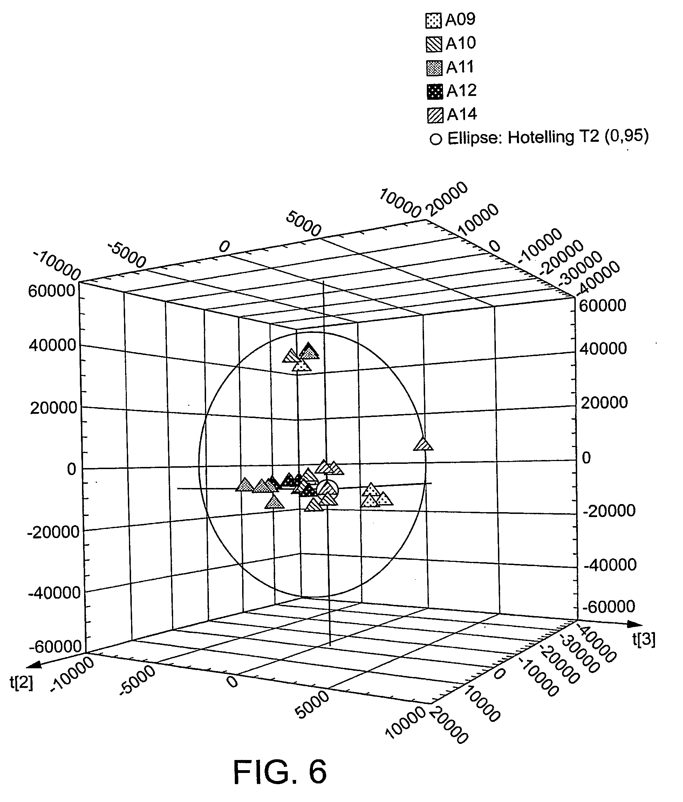

Figure 6 is a 3-D score plot of the bioconversion of animal fat. The results indicate relatively small differences between the inoculated and the control with respect to the animal fat.

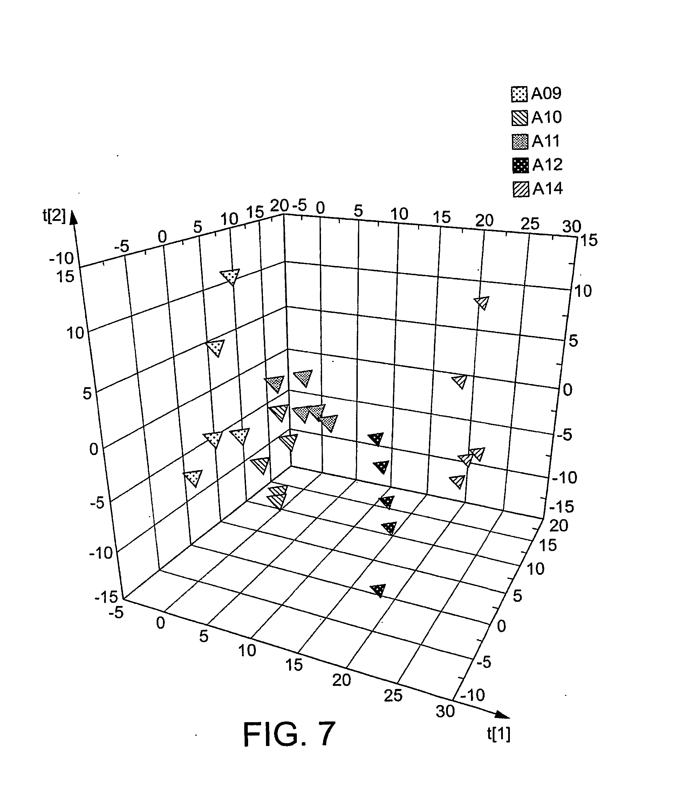

Figure 7 is an LC-MS spectrum at negative ionization, giving the differences in fatty acids. In contrast to figure 6, there is a change in fatty acid between the inoculated samples and the control. Inoculum A09 (L002) gives more fatty acid.

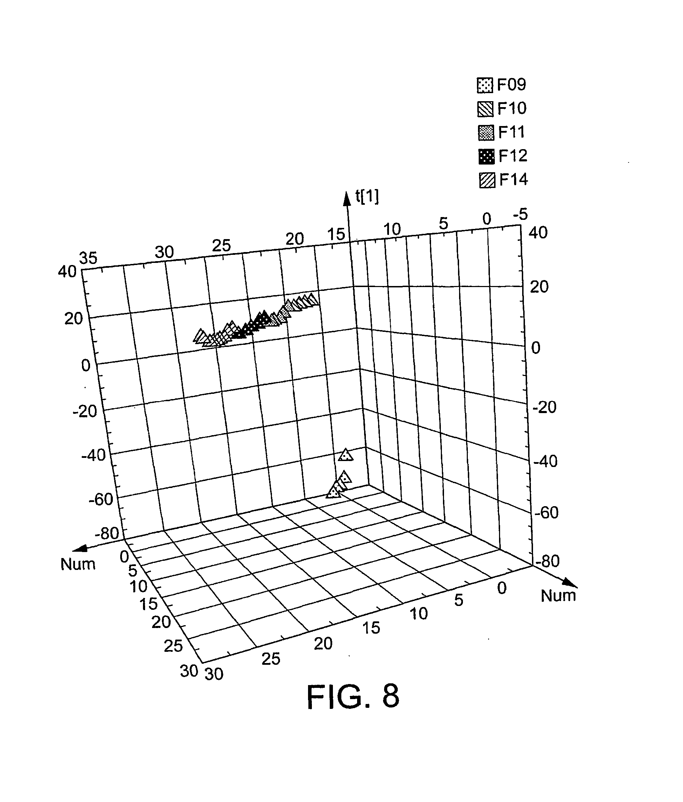

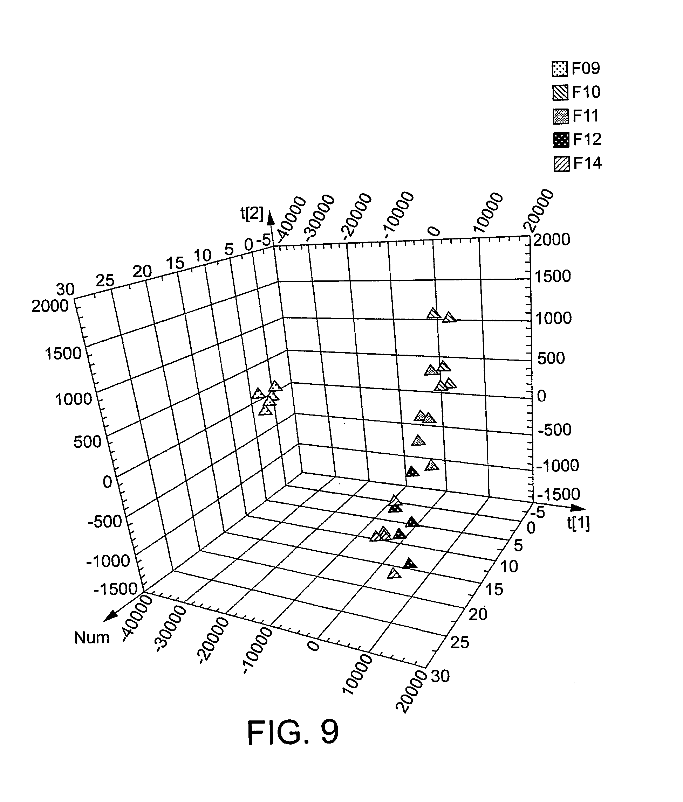

Figure 8 shows the bioconversion of soya oil. The inoculum corresponding to masterplate L002 gives the largest effect on the triglyceride composition. The corresponding results in figure 9 on fatty acids formed mirror this result although some changes with inoculum F10 (L004) are also observed.

Thus masterplate L002 has been shown to alter the fatty acid composition of an animal oil indicating that some or all of the microorganisms in masterplate L002 are acting to cleave the ester linkage in the glyceride molecule forming fatty acids. Glycerides in raw fish oil are rapidly converted to fatty acids by exposure to masterplate L019 and masterplate L004. Glycerides of refined fish oil are bioconverted by masterplate L004, masterplate L018 and masterplate L019. Glycerides of soya oil are bioconverted by masterplate L002.

Thus one or more microorganisms in the four different masterplates bioconverts triglycerides from a multiple of different raw material sources. Initial tests suggest *Geobacillus* micro-organisms may be instrumental in this degradation. Importantly, the examples also show that a mixture of different biooil feeds can be used at once and hence the eventual biofuel can contain a variety of different raw material sources.

## Claims

1. A process for the formation of biodiesel comprising the steps of:
(I) obtaining a biooil containing at least one fatty acid glyceride;
(II) contacting the biooil with at least one microorganism from the species *Geobacillus* or *Acinetobacter* capable of hydrolysing at least one ester bond of said at least one glyceride to form a hydrolysed biooil; and
(III) directly or indirectly using said hydrolysed biooil in biodiesel.

2. A process as claimed in claim 1, wherein said at least one microorganism is *Acinetobacter venetianus, Geobacillus pallidus, Geobacillus kaustrophilus, Geobaclllus toebli* or *Geobacillus thermoglucosidasius.*

3. A process as claimed in claim 1 or 2, wherein step (II) takes place at a temperature greater than 30 degrees C.

4. A process as claimed in any preceding claim, wherein said microorganism is A. *venetianus.*

5. A process as claimed in any preceding claim, wherein said microorganism is the *A. venetianus* strain deposited under the Budapest Treaty as DSM19446.

6. A process as claimed in any preceding claim, wherein the at least one fatty acid glyceride derives from an animal or fish source.

7. A process for the formation of biodiesel claimed in any preceding claim, wherein step (III) comprises:
(a) hydrotreating said hydrolysed biooil; optionally
(b) isomerising the hydrotreated blooil; and
(c) using the resulting product of step (a) or, if carried out, step (b) in biodiesel.

## Patentansprüche

1. Verfahren für die Erzeugung von Biodiesel, umfassend die Schritte:
(I) Gewinnen eines Bioöls, enthaltend mindestens ein Fettsäureglycerid;
(II) Inkontaktbringen des Bioöls mit mindestens einem Mikroorganismus von der Spezies *Geobacillus* oder *Acinetobacter,* fähig zum Hydrolysieren von mindestens einer Esterbindung des mindestens einen Glycerids, um ein hydrolysiertes Bioöl zu erzeugen; und
(III) direktes oder indirektes Verwenden des hydrolysierten Bioöls in Biodiesel.

2. Verfahren nach Anspruch 1, wobei der mindestens eine Mikroorganismus *Acinetobacter venetianus, Geobacillus pallidus, Geobacillus kaustrophilus, Geobacillus toebli* oder *Geobacillus thermoglucosidasius* ist.

3. Verfahren nach Anspruch 1 oder 2, wobei Schritt (II) bei einer Temperatur von mehr als 30 Grad C stattfindet.

4. Verfahren nach einem vorhergehenden Anspruch, wobei der Mikroorganismus A. *venetianus* ist.

5. Verfahren nach einem vorhergehenden Anspruch, wobei der Mikroorganismus der *A.-venetianus-Stamm,* hinterlegt nach dem Budapester Vertrag als DSM19446, ist.

6. Verfahren nach einem vorhergehenden Anspruch, wobei das mindestens eine Fettsäureglycerid aus einer Quelle von Tier oder Fisch stammt.

7. Verfahren für die Erzeugung von Biodiesel nach einem vorhergehenden Anspruch, wobei Schritt (III) umfasst:
(a) Hydrobehandeln des hydrolysierten Bioöls; wahlweise
(b) Isomerisieren des hydrobehandelten Bioöls; und
(c) Verwenden des resultierenden Produkts aus Schritt (a) oder, wenn ausgeführt, Schritt (b) in Biodiesel.

## Revendications

1. Procédé de formation de biodiesel comprenant les étapes consistant à:
(i) obtenir une bio-huile contenant au moins un glycéride d'acide gras,
(ii) mettre la bio-huile en contact avec au moins un microorganisme provenant de l'espèce *Geobacillus* ou *Acinetobacter,* capable d'hydrolyser au moins une liaison ester dudit au moins un glycéride pour former une bio-huile hydrolysée; et
(iii) utiliser directement ou indirectement ladite bio-huile hydrolysée dans du biodiesel.

2. Procédé selon la revendication 1, dans lequel le au moins un microorganisme est *Acinetobacter venetianus, Geobacillus pallidus, Geobacillus kaustrophilus, Geobacillus toebli* ou *Geobacillus thermoglucosidasius.*

3. Procédé selon la revendication 1 ou 2, dans lequel l'étape (ii) est effectuée à une température supérieure à 30°C.

4. Procédé selon l'une quelconque des revendications précédentes, dans lequel ledit microorganisme est *A. venetianus.*

5. Procédé selon l'une quelconque des revendications précédentes, dans lequel ledit microorganisme est la souche *A. venetianus* déposée aux termes du Traité de Budapest sous DSM19446.

6. Procédé selon l'une quelconque des revendications précédentes, dans lequel le au moins un glycéride d'acide gras est dérivé d'une source animale ou de poisson.

7. Procédé de formation de biodiesel selon l'une quelconque des revendications précédentes, dans lequel l'étape (iii) comprend:
(a) hydrotraiter ladite bio-huile hydrolysée; optionnellement
(b) isomériser la bio-huile hydrotraitée; et
(c) utiliser le produit obtenu de l'étape (a) ou, si effectuée, de l'étape (b) dans du biodiesel.
